# EUROPEAN PATENT SPECIFICATION

(11) **EP 2 730 577 B1**
(45) Date of publication and mention of the grant of the patent: **08.04.2015**
(21) Application number: 13748963.9
(22) Date of filing: 05.02.2013
(51) Int. Cl.: C07D 471/06, A61P 13/00, A61K 31/437

(54) **Crystal modification of 6-hydroxy-4-oxo-2,4-dihydro-1H-pyrrolo[3,2,1-ij]quinoline-5-carboxylic acid para-methoxyanilide**
Kristallmodifikation von 6-Hydroxy-4-oxo-2,4-dihydro-1H-pyrrolo[3,2,1-ij]chinolin-5-carbonsäure Para-methoxyanilid
Forme cristalline du para-méthoxyanilide de l'acide 6-hydroxy-4-oxo-2,4-dihydro-1H-pyrrolo[3,2,1-ij]quinoléine-5-carbonique

(30) Priority: 16.02.2012 RU 2012105420
(43) Date of publication of application: 14.05.2014
(73) Proprietor: Otkrytoe Aktsionernoe Obschestvo Mezhdunarodnaya, Moscow 123098 (RU)
(72) Inventor: UKRAINETS, Igor Vasilevich, 62442, Kharkovskaya obl. p/o Bolshaya Danilovka (UA)
(74) Representative: Jeck, Anton
(86) International application number: PCT/RU2013/000081
(87) International publication number: WO 2013/122509

(56) References cited:
- RU-C2- 2 269 517
- UA-C2- 86 286
- UA-C2- 86 883
- UKRAINETS I.V. ET AL.: '4-Hydroxy-2-quinolones 138*. Synthesis and study of structure-biological activity relationships in a series of 1-hydroxy-3-oxo-5,6-dihydro-3H-pyrrolo[3,2, 1-ij] quinoline-2-carboxylic acid anilides.' CHEMISTRY OF HETEROCYCLIC COMPOUNDS vol. 43, no. 12, 2007, pages 1532 - 1539, XP 002660338

## Description

The present invention relates to organic chemistry, more particularly, to a novel crystal modification of 6-hydroxy-4-oxo-2,4-dihydro-1H-pyrrolo[3,2,1-ij]-quinoline-5-carboxylic acid para-methoxyanilide, which can be used in the chemical-pharmaceutical industry and medicine.

A method for the crystallization from DMF used for the preparation of 6-hydroxy-4-oxo-2,4-dihydro-1H-pyrrolo[3,2,1-ij]-quinoline-5-carboxylic acid para-methoxyanilide represented in Formula 1, which exhibits a diuretic effect, is known in the art [1]. X-ray powder diffraction analysis data demonstrated that the product of said reaction is a pure polymorphic modification of the anilide (I) with a light yellow color, which is tentatively called the α-form. Said form is characterized by a certain set of values of interplanar spacing and relative intensity of reflection (Table 1).

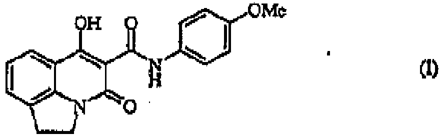

A disadvantage of the known crystal modification of 6-hydroxy-4-oxo-2,4-dihydro-1H-pyrrolo[3,2,1-ij]-quinoline-5-carboxylic acid para-methoxyanilide is that said compound is obtained in the form of fine needle-like crystals. This specific characteristic causes several significant technological problems in large-scale production: a precipitate of the fine α-form crystals is quite difficult to filter, wash, and dry. More difficulties arise from the crystallization with DMF: the high boiling point thereof hinders its removal from the final fine-crystal precipitate, while the high toxicity of said solvent puts very strict requirements on the allowed content thereof in pharmaceutical compositions (the maximum allowed residual amount in pharmaceutical substances should not exceed 880 ppm [2]).

**Table 1**

| Interplanar spacings (d) and relative intensities of reflections (Iᵣₑₗ) of the known crystal modification of 6-hydroxy-4-oxo-2,4-dihydro-1H-pyrrolo[3,2,1-ij]-quinoline-5-carboxylic acid para-methoxyanilide (α-form) | | | | | |
|---|---|---|---|---|---|
| d, Å | Iᵣₑₗ | d, Å | Iᵣₑₗ | d, Å | Iᵣₑₗ |
| 10,415 | 67 | 2,890 | 1 | 2,227 | 1 |
| 8,897 | 14 | 2,809 | 1 | 2,185 | 1 |
| 6,442 | 100 | 2,773 | 1 | 2,151 | <1 |
| 5,926 | <1 | 2,736 | 1 | 2,104 | 1 |
| 5,648 | 2 | 2,690 | 1 | 2,087 | 1 |
| 5,382 | 5 | 2,613 | 1 | 2,071 | 1 |
| 5,256 | 12 | 2,603 | 1 | 2,054 | 1 |
| 4,949 | 4 | 2,578 | 1 | 1,988 | 1 |
| 4,758 | 10 | 2,516 | 1 | 1,983 | 1 |
| 4,476 | <1 | 2,506 | 1 | 1,952 | 1 |
| 4,179 | 29 | 2,489 | 1 | 1,939 | 1 |
| 4,036 | 2 | 2,463 | 1 | 1,880 | 1 |
| 3,906 | <1 | 2,448 | 1 | 1,859 | 1 |
| 3,773 | 1 | 2,440 | 1 | 1,824 | 1 |
| 3,667 | 4 | 2,413 | <1 | 1,810 | 1 |
| 3,401 | 98 | 2,369 | 1 | 1,758 | <1 |
| 3,252 | 22 | 2,361 | 1 | 1,721 | 2 |
| 3,147 | 7 | 2,349 | 1 | 1,698 | 1 |
| 2,962 | 1 | 2,329 | 1 | 1,678 | 1 |
| 2,923 | 1 | 2,276 | <1 | 1,673 | <1 |

The objective of the present invention is to prepare a novel crystal modification of 6-hydroxy-4-oxo-2,4-dihydro-1H-pyrrolo[3,2,1-ij]-quinoline-5-carboxylic acid para-methoxyanilide with more advantageous pharmaceutical and biological properties.

Said objective is achieved by the preparation of a novel crystal modification of the anilide (I), tentatively called the β-form, characterized by different values of Interplanar spacings and relative intensities of the reflections (Table 2), which also exhibits an enhanced diuretic effect.

**Table 2**

| Interplanar spacings (d) and relative intensities of reflections (Iᵣₑₗ) of the novel crystal modification of 6-hydroxy-4-oxo-2,4-dihydro-1H-pyrrolo[3,2,1-ij]-quinoline-5-carboxylic acid para-methoxyanilide (β-form) | | | | | |
|---|---|---|---|---|---|
| d, Å | Iᵣₑₗ | d, Å | Iᵣₑₗ | d, Å | Iᵣₑₗ |
| 15,189 | 6 | 3,868 | 1 | 2,776 | <1 |
| 11,176 | 54 | 3,753 | 4 | 2,740 | <1 |
| 7,912 | 5 | 3,728 | 10 | 2,728 | 1 |
| 7,821 | 10 | 3,694 | 52 | 2,718 | 1 |
| 7,750 | 13 | 3,666 | 10 | 2,656 | 1 |
| 7,634 | 4 | 3,650 | 4 | 2,642 | 1 |
| 7,426 | 28 | 3,600 | 4 | 2,630 | <1 |
| 7,376 | 39 | 3,575 | 8 | 2,601 | <1 |
| 6,751 | 18 | 3,546 | 42 | 2,585 | 1 |
| 6,674 | 36 | 3,520 | 7 | 2,576 | <1 |
| 6,027 | <1 | 3,475 | <1 | 2,494 | <1 |
| 5,867 | 4 | 3,442 | 1 | 2,482 | 1 |
| 5,798 | 11 | 3,417 | <1 | 2,475 | 1 |
| 5,718 | 21 | 3,388 | 2 | 2,446 | <1 |
| 5,648 | 15 | 3,362 | 24 | 2,412 | <1 |
| 5,609 | 10 | 3,339 | 100 | 2,402 | 1 |
| 5,561 | 3 | 3,323 | 31 | 2,312 | <1 |
| 5,084 | 4 | 3,257 | 2 | 2,301 | <1 |
| 4,907 | 1 | 3,240 | 3 | 2,295 | <1 |
| 4,856 | <1 | 3,220 | 2 | 2,283 | <1 |
| 4,751 | 2 | 3,163 | 1 | 2,273 | 1 |
| 4,714 | 5 | 3,145 | 2 | 2,267 | <1 |
| 4,681 | 7 | 3,108 | 17 | 2,247 | <1 |
| 4,660 | 6 | 3,083 | 2 | 2,236 | 1 |
| 4,589 | 1 | 3,071 | <1 | 2,227 | <1 |
| 4,567 | 3 | 3,049 | <1 | 2,221 | <1 |
| 4,548 | 5 | 2,966 | <1 | 2,166 | <1 |
| 4,366 | <1 | 2,955 | 1 | 2,113 | <1 |
| 4,225 | 1 | 2,944 | 1 | 2,103 | 2 |
| 4,187 | 4 | 2,933 | <1 | 2,095 | <1 |
| 4,168 | 8 | 2,869 | <1 | 2,086 | <1 |
| 4,140 | 13 | 2,854 | <1 | 2,072 | <1 |
| 4,100 | 4 | 2,840 | <1 | 2,051 | <1 |
| 4,083 | 2 | 2,831 | 1 | 2,043 | 1 |
| 4,047 | 1 | 2,813 | 1 | 2,037 | <1 |
| 3,992 | 1 | 2,806 | 1 | 2,021 | <1 |
| 3,977 | 4 | 2,793 | 2 | 1,880 | <1 |
| 3,946 | 12 | 2,783 | 1 | 1,874 | <1 |
| 3,907 | 7 | | | | |

Unlike the α-form, the novel polymorphic modification of 6-hydroxy-4-oxo-2,4-dihydro-1H-pyrrolo[3,2,1-ij]-quinoline-5-carboxylic acid para-methoxyanilide (β-form) consists of large light yellow colored octahedral crystals, which are easily filtered and washed. Drying the β-form also does not present a problem because it is prepared by crystallization of the anilide (I) from the low-boiling and low-toxic ethyl acetate (the maximum allowed residual amount in pharmaceutical substances is 5000 ppm [2]).

Several various physical, chemical, and biological methods of analysis carried out in similar conditions were used to characterize both polymorphic modification of 6-hydroxy-4-oxo-2,4-dihydro-1H-pyrrolo[3,2,1-ij]-quinoline-5-carboxylic acid para-methoxyanilide.

### Example 1. X-ray powder diffraction analysis.

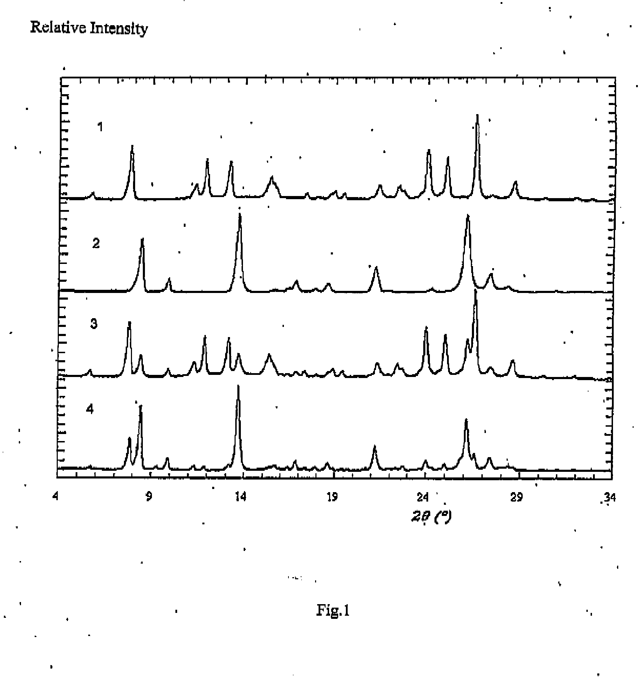

To prove that the known and the novel product are different pure polymorphic modifications of the anilide (I), an X-ray powder diffraction analysis was conducted for the individual α- and β-forms as well as for the mixtures thereof that resulted from recrystallization from other solvents. The study was carried out on a Siemens D500 diffractometer (using CuKa radiation and graphite monochromator). The obtained X-ray powder patterns were analyzed with PowderX and WinPLOTR software [3,4] and are shown on Fig.1.

Data shown on Fig.1 clearly demonstrate that X-ray powder patterns of α- and β-forms have nothing in common. Therefore, said samples are pure polymorphic modifications of the anilide (I). At the same time, the samples obtained by crystallization from dioxane and ethanol are mixtures of said two polymorphs existing in different ratios, because their corresponding X-ray powder patterns contain bands characteristic of both α- and β-forms. Samples 3 and 4 are obtained from different solvents; therefore, they cannot be crystal solvates. The same statement holds true for sample 2 (α-form). An attempt to decipher the structure of the α-form from X-ray powder patterns was futile because X-ray powder pattern bands were distinctly widened, and therefore, cell parameters could not be reliably identified. α-form crystals, which would be suitable for a single crystal X-ray diffraction study, also could not be obtained. Remarkably, said polymorphic modification is likely to have an elementary cell volume of 9000 A³, and in such case, a structural determination based on X-ray powder patterns is hardly possible. However, both X-ray powder patterns themselves (Fig.1) and the data on interplanar spacings and relative intensities of the reflections characteristic of each of the α- and p- polymorphic modifications of 6-hydroxy-4-oxo-2,4-dihydro-1H-pyrrolo[3,2,1-ij]-quinoline-5-carboxylic acid para-metoxyanilide (Tables 1 and 2) can be used for a reliable identification of said modifications.

Fig.1. X-ray powder patterns of anilide (I) samples obtained by crystallization from ethyl acetate (1 - β-form), DMF (2 - α-form), dioxane (3 - mixture of α- and β-forms with the β-form predominantly), and ethanol (4 - mixture of α- and β-forms with the α - form predominantly).

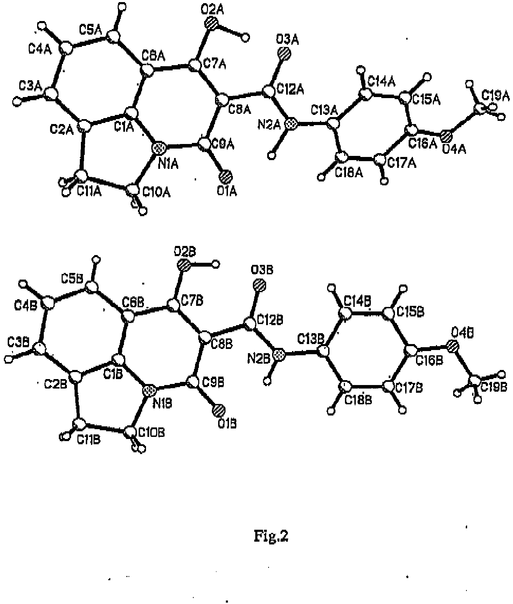

Example 2. Single crystal X-ray diffraction study. Crystals of the novel β-form of 6-hydroxy-4-oxo-2,4-dihydro-1H-pyrrolo[3,2,1-ij]-quinoline-6-carboxylic acid para-methoxyanilide were suitable for a single crystal X-ray diffraction study, which allowed for determination of the unique spatial structure thereof. Thus, in particular, the independent part of the elementary cell was determined to have two molecules (A and B), which differed in the methoxy group orientation and in the degree of flatness (Fig. 2, Tables 3 and 4). The tricyclic moiety, O(1) and O(2) atoms as well as the car bamide moiety in both molecules are located in one plane with 0.04 Å accuracy, which could probably be attributed to the presence of two strong intramolecular hydrogen bonds O(2)-H(20)...O(3) [H...O 1.30 Å, O-H...O 171° in molecule A and H...O 1.56 Å, O-H...O 151° in molecule B] and N(2)-H(2N)...O(1) [H...O 1.61 Å, N-H...O 144° in A and H...O 1.80 Å, N-H...O 147° in B]. The hydrogen bond formation also causes a significant increase in the length of the O(1)-C(9) bond to 1.244(1) A in A and to 1.252(2) A in B, as well as the O(3)-C(12) bond to 1.268(2) A In A and 1.255(2) A in B as compared to their mean value [5] of 1.210 Å. On the contrary, the O(2)-C(7) bond is shortened to 1.359(2) A in A and to 1.329(2) A in B (mean value is 1.362 A). The C(7)-C(8) bond is lengthened to 1.372(2) Å in A and to 1.391 (2) A in B (1.326 Å mean value), which is typical of quinolone compounds. The para-methoxyphenyl substituent is in the ap-conformation relative to the C(8)-C(12) bond, and in the A molecule, it is somewhat unfolded relative to the carbamide moiety plane, while in the B molecule, it is coplanar to said plane (dihedral angles: C(13)-N(2)-C(12)-C(8) 176.3(1)° in A and -178.5(2)° in B, C(12)-N(2)-C(13)-C(14) 22.4(2)° in A and 0.6(3)° in B). The alignment of the carbamide moiety and the aromatic ring against each other is affected by two opposite factors: the intramolecular hydrogen bond C(14)-H(14)...O(3) [H...O 2.38 Å C-H...O 117° in molecule A and H...O 2.28 Å C-H...O 121° in molecule B], which stabilizes the coplanar position of the moieties, and the repulsion between H(18)... H(2N) 2.36 Å hydrogen atoms in A and 2.30 Å in B (the sum of Van der Waals radiuses [6] 2.34 A), which aids in the unfolding of said moieties against each other. It can be contemplated that the more pronounced coplanarity of the carbamide moiety and the aromatic ring in molecule B is caused by the stronger effect of hydrogen binding.

Fig. 2. A and B molecular structure of the β-form of 6-hydroxy-4-oxo-2,4-dihydro-1H-pyrrolo[3,2,1-ij]-quinoline-5-carboxylic acid para-methoxyanilide with atom numbering.

The A molecule's methoxy group is in the cis-position relative to the C(15)-C(16) bond and in the trans-position relative to said bond in the B molecule (dihedral angle is C(15)-C(16)-O(4)-C(19) 1.6(2)° in A, and 172.7(1)° in B) and is practically coplanar to the aromatic ring's plane despite of the rather strong repulsion between the ring atoms and the methyl group (shortened intramolecular contacts H(15)...C(19) 2.56 Å (2.87 A), H(19a)...C(15) 2.78 Å (2.87 A), H(19c)...C(15) 2.77 Å (2.87 A) in molecule A and H(17)...C(19) 2.56 Å (2.87 A), H(17)...H(19d) 2.32 Å (2.34 A), H(19d)...C(17) 2.78 Å (2.87 A), H(19e)...C(17) 2.77 Å (2.87 A) in molecule B).

Layers formed in the crystals of molecules A and B are parallel to the crystallographic plane (-1 -1 2). The molecules of the adjoining layers are situated in a "head to tail" configuration relative to one another, while the presence of stacking interaction can be contemplated based on the overlapping degree of said molecules and the distance between the layers (3.37 Å)

**Table 3**

| Bond lengths (l) in the β-form structure of 6-hydroxy-4-oxo-2,4-dihydro-1H-pyrrolo[3,2,1-ij]-quinoline-5-carboxylic acid para-methoxyanilide | | | |
|---|---|---|---|
| Bond | I, Å | Bond | I, Å |
| O(1A)-C(9A) | 1.244(1) | O(2A)-C(7A) | 1.359(2) |
| O(3A)-C(12A) | 1.268(2) | O(4A)-C(16A) | 1.376(2) |
| O(4A)-C(19A) | 1.410(2) | N(1A)-C(1A) | 1.366(2) |
| N(1A)-C(9A) | 1,370(2) | N(1A)-C(10A) | 1.484(2) |
| N(2A)-C(12A) | 1,322(2) | N(2A)-C(13A) | 1.419(2) |
| C(1A)-C(6A) | 1.365(2) | C(1A)-C(2A) | 1.398(2) |
| C(2A)-C(3A) | 1.348(2) | C(2A)-C(11A) | 1.501(2) |
| C(3A)-C(4A) | 1.410(2) | C(4A)-C(5A) | 1.373(2) |
| C(5A)-C(6A) | 1.406(2) | C(6A)-C(7A) | 1.415(2) |
| C(7A)-C(8A) | 1.372(2) | C(8A)-C(9A) | 1.469(2) |
| C(8A)-C(12A) | 1.481(2) | C(10A)-C(11A) | 1.547(2) |
| C(13A)-C(14A) | 1.369(2) | C(13A)-C(18A) | 1.388(2) |
| C(14A)-C(15A) | 1.398(2) | C(15A)-C(16A) | 1.371(2) |
| C(16A)-C(17A) | 1.379(2) | C(17A)-C(18A) | 1.374(2) |
| O(1B)-C(9B) | 1.252(2) | O(2B)-C(7B) | 1.329(2) |
| O(3B)-C(12B) | 1.255(2) | O(4B)-C(16B) | 1.368(2) |
| O(4B)-C(19B) | 1.422(2) | N(1B)-C(9B) | 1.358(2) |
| N(1B)-C(1B) | 1.365(2) | N(1B)-C(10B) | 1.466(1) |
| N(2B)-C(12B) | 1.345(2) | N(2B)-C(13B) | 1.408(2) |
| C(1B)-C(6B) | 1.372(2) | C(1B)-C(2B) | 1.389(2) |
| C(2B)-C(3B) | 1.364(2) | C(2B)-C(11B) | 1.504(2) |
| C(3B)-C(4B) | 1.396(2) | C(4B)-C(5B) | 1.380(2) |
| C(5B)-C(6B) | 1.409(2) | C(6B)-C(7B) | 1.424(2) |
| C(7B)-C(8B) | 1.391(2) | C(8B)-C(9B) | 1.460(2) |
| C(8B)-C(12B) | 1.478(2) | C(10B)-C(11B) | 1.551(2) |
| C(13B)-C(14B) | 1.380(2) | C(13B)-C(14B) | 1.385(2) |
| C(14B)-C(15B) | 1.377(2) | C(15B)-C(16B) | 1.381(2) |
| C(16B)-C(17B) | 1.372(2) | C(17B)-C(18B) | 1.378(2) |

**Table 4**

| Bond angles (ω) in the β-form structure of 6-hydroxy-4-oxo-2,4-dihydro-1H-pyrrolo[3,2,1-ij]-quinoline-5-carboxylic acid para-methoxyanilide | | | |
|---|---|---|---|
| Bond angle | ω, degree | Bond angle | ω, degree |
| C(16A)-O(4A)-C(19A) | 117.9(1) | C(1A)-N(1A)-C(9A) | 123.9(1) |
| C(1A)-N(1A)-C(10A) | 111.1(1) | C(9A)-N(1A)-C(10A) | 125.0(1) |
| C(12A)-N(2A)-C(13A) | 128.6(2) | C(6A)-C(1A)-N(1A) | 123.5(2) |
| C(6A)-C(1A)-C(2A) | 125.1(2) | N(1A)-C(1A)-C (2A) | 111.4(2) |
| C(3A)-C(2A)-C(1A) | 117.5(2) | C(3A)-C(2A)-C(11A) | 133.6(2) |
| C(1A)-C(2A)-C(11A) | 108.9(1) | C(2A)-C(3A)-C(4A) | 119.7(2) |
| C(5A)-C(4A)-C(3A) | 121.7(2) | C(4A)-C(5A)-C(6A) | 119.5(2) |
| C(1A)-C(6A)-C(5A) | 116.5(2) | C(1A)-C(6A)-C(7A) | 115.2(2) |
| C(5A)-C(6A)-C(7A) | 128,3(2) | O(2A)-C(7A)-C(8A) | 120.4(2) |
| O(2A)-C(7A)-C(6A) | 116.8(2) | C(8A)-C(7A)-C(6A) | 122.8(2) |
| C(7A)-C(8A)-C(9A) | 120.5(2) | C(7A)-C(8A)-C(12A) | 119.9(2) |
| C(9A)-C(8A)-C(12A) | 119.7(2) | O(1A)-C(9A)-N(1A) | 119.7(2) |
| O(1A)-C(9A)-C(8A) | 126.2(2) | N(1A)-C(9A)-C(8A) | 114.1(1) |
| N(1A)-C(10A)-C(11A) | 103.9(1) | C(2A)-C(11A)-C(10A) | 104.6(1) |
| O(3A)-C(12A)-N(2A) | 122.6(2) | O(3A)-C(12A)-C(8A) | 119.6(2) |
| N(2A)-C(12A)-C(8A) | 117.8(2) | C(14A)-C(13A)-C(18A) | 118.4(2) |
| C(14A)-C(13A)-N(2A) | 125.7(2) | C(18A)-C(13A)-C(2A) | 115.9(2) |
| C(13A)-C(14A)-C(15A) | 120.9(2) | C(16A)-C(15A)-C(14A) | 119.9(2) |
| C(15A)-C(16A)-O(4A) | 125.8(2) | C(15A)-C(16A)-C(17A) | 119.6(2) |
| O(4A)-C(16A)-C(17A) | 114.6(2) | C(18A)-C(17A)-C(16A) | 120.2(2) |
| C(17A)-C(18A)-C(13A) | 121.1(2) | C(16B)-C(4B)-C(19B) | 117.5(1) |
| C(9B)-N(1B)-C(1B) | 123.3(2) | C(9B)-N(1B)-C(10B) | 125.1(1) |
| C(1B)-N(1B)-C(10B) | 111.7(1) | C(12B)-N(2B)-C(13B) | 129.7(2) |
| N(1B)-C(1B)-C(6B) | 123.6(2) | N(1B)-C(1B)-C(2B) | 118.2(2) |
| C(6B)-C(1B)-C(2B) | 124.6(2) | C(3B)-C(2B)-C(1B) | 117.5(2) |
| C(3B)-C(2B)-C(11B) | 134.3(2) | C(1B)-C(2B)-C(11B) | 108.2(2) |
| C(2B)-C(3B)-C(4B) | 120.4(2) | C(5B)-C(4B)-C(3B) | 121.0(2) |
| C(4B)-C(5B)-C(6B) | 119.8(2) | C(1B)-C(6B)-C(5B) | 116.7(2) |
| C(1B)-C(6B)-C(7B) | 115.7(2) | C(5B)-C(6B)-C(7B) | 127.6(2) |
| O(2B)-C(7B)-C(8B) | 120.9(2) | O(2B)-C(7B)-C(6B) | 117.7(2) |
| C(8B)-C(7B)-C(6B) | 121.4(2) | C(7B)-C(8B)-C(9B) | 120.3(2) |
| C(7B)-C(8B)-C(12B) | 118.7(2) | C(9B)-C(8B)-C(12B) | 121.0(2) |
| O(1B)-C(9B)-N(1B) | 119.7(2) | O(1B)-C(9B)-C(8B) | 124.6(2) |
| N(1B)-C(9B)-C(8B) | 115.6(2) | N(1B)-C(10B)-C(11B) | 103.5(1) |
| C(2B)-C(11B)-C(10B) | 104.9(1) | O(3B)-C(12B)-N(2B) | 122.0(2) |
| O(3B)-C(12B)-C(8B) | 120.4(2) | N(2B)-C(12B)-C(8B) | 117.6(2) |
| C(18B)-C(13B)-C(14B) | 118.2(2) | C(18B)-C(13B)-N(2B) | 117.2(2) |
| C(14B)-C(13B)-N(2B) | 124.7(2) | C(15B)-C(14B)-C(13B) | 119.5(2) |
| C(14B)-C(15B)-C(16B) | 122.3(2) | O(4B)-C(16B)-C(17B) | 126.0(2) |
| O(4B)-C(16B)-C(15B) | 116.0(2) | C(17B)-C(16B)-C(15B) | 118.0(2) |
| C(16B)-C(17B)-C(18B) | 120.3(2) | C(17B)-C(18B)-C(3B) | 121.8(2) |

β-form crystals of 6-hydroxy-4-oxo-2,4-dihydro-1H-pyrrolo[3,2,1-ij]-quinoline-5-carboxylic acid para-methoxyanilide are monoclinic (ethyl acetate) at 20°C a= 16.381(2), b = 8.459(1), c = 23.630(2) Å, β = 108.61(1)°, V = 3103.1(4) Å³, M_{T}= 336.34, Z = 8, space group P2ₗ/n, d cal.= 1.440 g/cm³, µ(MoK_{α}) = 0.103 mm⁻¹, F(000) = 1408. Parameters of the elementary cell and the intensity of 33430 reflections (8993 independent, Rᵢₙₜ = 0.095) were measured on an Xcalibur-3 diffractometer (MoKα emission, CCD-detector, graphite monochromator, ω-scaning, 2Θₘₐₓ= 60°).

The structure was deciphered by the direct method using SHELXTL [7] software. Hydrogen atom locations were determined by differential synthesis of the electron density and confirmed by the "riding" model with Uᵢₛₒ= nU_{eq} of the non-hydrogen atom connected to said hydrogen atom (n = 1.5 for the methyl group and n = .2 for the remaining hydrogen atoms). Hydrogen atoms forming hydrogen bonds are confirmed by isotropic approximation. The structure is confirmed according to F² by the full matrix least-squares method in the anisotropic approximation for non-hydrogen atoms to wR₂ = 0.032 according to 8873 reflections (R₁ =0.032 according to 1951 reflections with F > 4σ (F), S = 0.465). The interatomic interplanar spacings and bond angles are shown in Tables 3 and 4, respectively.

Example 3. Liquid-state NMR spectroscopy. Qualitative analysis of the known α-form and the novel β-form of 6-hydroxy-4-oxo-2,4-dihydro-1H-pyrrolo[3,2,1-ij]-quinoline-5-carboxylic acid para-methoxyanilide was carried out bv liquid-state ¹H and ¹³C NMR spectroscopy.

'H and ¹³C NMR spectra were recorded on a Varian Mercury-400 (400 MHz for ¹H and 100 MHz for ¹³C) in a DMSO-d₆ solution using TMS as an internal standard.

Data shown in Tables 5 and 6 demonstrate that both test samples have identical chemical formulas, i.e. they are 6-hydroxy-4-oxo-2,4-dihydro-1H-pyrrolo[3,2,1-ij]-quinoline-5-carboxylic acid para-methoxyanilides.

As expected, once dissolved, dissimilar polymorphic modifications, which are characteristic of crystals, disappear, and as a result, the known α-form and the novel β-form yield practically identical ¹H and ¹³C NMR spectra.

**Table 5**

| Chemical shifts of the ¹H nuclei in the initial α-form and the novel β-form of the anilide (I) in ¹H NMR spectra In a DMSO-d₆ solution | | |
|---|---|---|
| Functional group | initial α-form | Novel β-form |
| OH (1H, s) | 16.56 | 16.48 |
| NH (1H, s) | 12.43 | 12.35 |
| H-7 (1H, d) | 7.67 | 7.58 |
| H-9,2',6' (3H, m) | 7.54 | 7.48 |
| H-8 (1H, t) | 7.23 | 7.16 |
| H-3',5' (2H, d) | 6.95 | 6.90 |
| CH₂-2 (2H, t) | 4.30 | 4.23 |
| OCH₃ (3H, s) | 3.77 | 3.75 |
| CH₂-1 (2H, t) | 3.38 | 3.30 |

**Table 6**

| Chemical shifts of the ¹³C nuclei in the initial α-form and the novel β-form of the anilide (I) in ¹³C NMR spectra in a DMSO-d₆ solution | | |
|---|---|---|
| Carbon atom | Initial α-form | Novel β-form |
| 6-C-OH | 172.66 | 172.57 |
| CONH | 169.58 | 169.50 |
| 4-C=O | 161.29 | 161.21 |
| 4'-COMe | 157.29 | 157.24 |
| 9b-C | 142.73 | 142.63 |
| 9-C | 132.42 | 132.24 |
| 1'-C | 130.68 | 130.72 |
| 7-C | 129.21 | 129.04 |
| 8-C | 124.27 | 124.14 |
| 2',6'-C | 123.02 | 122.94 |
| 6a-C | 121.02 | 120.97 |
| 3',5'-C | 115.14 | 115.10 |
| 9a-C | 112.82 | 112.79 |
| 5-C | 98.23 | 98.17 |
| OCH₃ | 56.10 | 56.07 |
| 2-CH₂ | 47.78 | 47.68 |
| 1-CH₂ | 27.29 | 27.25 |

Example 4. High-performance liquid chromatography. Purity of α- and β-forms of 6-hydroxy-4-oxo-2,4-dihydro-1H-pyrrolo[3,2,1-ij]-quinoline-5-carboxylic acid para-methoxyanilide was determined by high-performance liquid chromatography (HPLC). The study was conducted on a Waters Alliance 2695 liquid chromatograph using a Waters PDA 2998 detector (diode matrix in the UV/VIS region), detector wavelength-235 nm, Agilent Zorbax SB-CN analytical column 250 x 4.6 mm, particle size - 5 µm, column thermostat temperature - 30°C, mobile phase: a 55:45 mixture of 0.05M phosphate buffer solution with pH 3.0 and acetonitrile, mobile phase flow rate - 1.0 ml/min. sample solution: 0.5 mg/ml in acetonitrile, sample volume of the sample solution - 20 µm.

**Table 7**

| Chromatographic retention times (RT) and peak areas (%) on the chromatograms of the initial α-form and the novel β-form of the anilide (I) | | |
|---|---|---|
| Sample | RT, min | Peak area, % |
| α-form | 16.914 | 99.93 |
| β-form | 17.375 | 100.0 |

Data in Table 7 demonstrate high purity of both known α-form and the novel β-form of 6-hydroxy-4-oxo-2,4-dihydro-1H-pyrrolo[3,2,1-ij]-quinoline-5-carboxylic acid para-methoxyanilide, wherein the main substance content is at least 99.93%.

Example 5. Solid-state NMR. Unlike liquid-state NMR, solid-state NMR is very well suited for determining the phase composition (including polymorphism) and structure of solids with any degree of order. Thus, solid-state ¹³C NMR was used in the study of polymorphic variants and polymorphic purity of the known α-form and the novel β-form of the anilide (I).

¹³C NMR spectra of both chemical modifications of the anilide (I) were obtained on an NMR AVANCE-II 400 BRUKER spectrometer, operational frequency 100.4 MHz, for ¹³C. The spectra were registered with a two-channel solid-state recorder on a MAS system (magic-angle spinning) with the rotor's outer diameter of 4 mm. The sample in the form of a powder was placed into ceramic MAS-rotors. The RPM of the sample was 12,000 Hz. MAS ¹³C NMR spectra were recorded using the linear ramp pulse cross polarization technique (CP/MAS) for ¹H during cross polarization employing thigh power radio frequency SW-TPPM (τ = δµε, ϕ = 1°) for protons during recording of the spectra. Contact time was 2 msec., time between scans was 2 sec., and number of scans was 1024. TMS (0ppm) was used as an external standard of the chemical shift scale.

**Table 8**

| Chemical shifts of the ¹³C nuclei in the initial α-form and the novel β-form of the anilide (I) In soild-state ¹³C NMR spectra | | |
|---|---|---|
| Carbon atom | Initial α-form | Novel β-form |
| 6-C-OH | 172.4 | 172.3 s |
| CONH | 167.6 | 168.1 d |
| 4-C=O | 160.2 | 161.0 s |
| 4'-COMe | 157.8 | 156.3 d |
| 9b-C | 141.9 | 142.2 s |
| 9-C | 131.7 | 132.0 d |
| 1'-C | 130.7 | 131.1 d |
| 7-C | 128.0 | 129.9s |
| 8-C | 124.0 | 128.1 d |
| 2',6'-C | 123.2 | 124.8 d |
| | 121.9 | 123.0 s |
| 6a-C | 120.7 | 121.6 d |
| 3',5-C | 118.9 | 116.6 s |
| | 112.5 | 114.3 d |
| 9a-C | 110.6 | 110.8 d |
| 5-C | 96.2 | 98.3 d |
| OCH₃ | 56.1 | 54.7 d |
| 2-CH₂ | 48.5 | 48.7 d |
| 1-CH₂ | 28.2 | 28.6 d |

Analysis of the data presented in Table 8 demonstrates that the novel β-form is a crystal phase, wherein the molecules of the initial compound exist in two nonequivalent positions (this conclusion fully concurs with the results described in Example 2 for the single crystal X-ray diffraction study of the β-form). Said conclusion was reached based on the doubling of the majority of ¹³C NMR signals in comparison to the ¹³C NMR spectra of said compound obtained in a liquid phase. The short bandwidth of the MAS ¹³C NMR spectra points to the high crystallinity of the substance.

In contrast, the known α-form is a polymorphic modification comprising only one type of molecule. That was indicated by the total number of singlet ¹³C NMR signals, the total of 19, which corresponds to the total number of carbon atoms In the study molecule; in addition, there was no evidence of singlet splitting as was observed for the β-form. Unlike liquid-state ¹³C NMR spectra of said substance, all carbon atoms in the crystal α-form are nonequivalent because the nonequivalence thereof is determined not only by their location within the molecule, but also by the position of the molecule itself relative to the adjoining molecules.

Band locations on the ¹³C NMR spectra of α- and β-forms do not match. The shorter bandwidth on the ¹³C NMR spectra of the β-form points to the higher degree of crystallinity thereof as compared to the α-form.

The study of the known α-form and the novel β-form by solid-phase ¹³C NMR spectroscopy revealed that said forms are distinctly different polymorphic modifications of 6-hydroxy-4-oxo-2,4-dihydro-1H-pyrrolo[3,2.1-ij]-quinoline-5-carboxylic acid para-methoxyanilide, effectively, with full polymorphic purity.

Example 6. Diuretic effect of the novel crystal modification (β-form) of 6-hydroxy-4-oxo-2,4-dihydro-1H-pyrrolo[3,2,1-ij]-quinoline-5-carboxylic acid para-methoxyanilide compared to the effect of the known crystal modification (α-form) of the same compound was studied on white rats weighing 180 - 200 g (12 animals per each compound) using a standard technique [8]. All tested animals received water via a gastric tube at 5 ml per 100 mg of body weight. The control group received the same amount of water with Tween 80. The tested substances were administered orally at 10 mglkg (median effective dose of the known α-form) as a fine suspension in water stabilized with Tween-80. After that, the tested animals were placed into "exchange cages". The urinary excretion intensity was determined by the amount of urine excreted by the animals over a period of 5 hrs. The experimental data shown in Table 9 leads to a conclusion that the diuretic action of the novel crystal modification (β-form) of 6-hydroxy-4-oxo-2,4-dihydro-1H-pyrrolo[3,2,1-ij]-quinoline-5-carboxylic acid para-methoxyanilide exceeds that of the known α-form by an average of 19%.

**Table 9**

| Diuretic effect of the known α-form and the novel β-form of the anilide (I) at the 10 mg/kg dose | | |
|---|---|---|
| Compound | Diuresis after 5 hr., ml | Diuretic effect, % |
| Known α-form | 9.7 ± 0.28 | 226 |
| Novel β-form | 10.5 ± 0.27 | 245 |
| Control | 4.3 ± 0.20 | 100 |

Thus, the disclosed herein complex of physical and chemical tests confirms that the claimed crystal modification (β-form) of 6-hydroxy,4-oxo-2,4-dihydro-1H-pyrrolo[3,2,1-ij]-quinoline-5-carboxylic acid para-methoxyanilide is a novel crystal modification. Unlike the known α-form, the obtained substance possesses superior pharmaceutical properties and exhibits an enhanced diuretic effect, which will allow for said substance to be widely used in various areas of medicine.

### References

1. Ukrainian Patent 86883 / C07D 215/22, A61 31/47, published 2009.
2. European Pharmacopoeia, 6th Edition. - 2008, Vol. 1. - P. 601-610.
3. C. Dong / PowderX: Windows-95-based program for powder X-ray dif-fraction data processing. - J. Appl. Crystallogr. - 1999. - Vol. 32. - P. 838.
4. J. Rodriguez-Carvajal, T. Roisnel / FullProf.98 and WinPLOTR: New Windows 95/NT Applications for Diffraction. Commission for Powder Diffraction, International Union of Crystallography, Newsletter No. 20 (May- August) Summer 1998.
5. H.-B. Burgi, J.D. Dunitz / Structure Correlation, VCH, Weinheim, 1994, Vol. 2. - P.741.
6. Yu.V. Zephirov/ Kristallographia. - 1997. Volume 42, #5. - p. 936
7. G. M. Sheldrick / Acta Crystallogr. - 2008. Vol. A64. - P. 112.
8. L.N. Sernov, V.V. Gatsura/ Elements of Experimental Pharmacology. M. Meditsina, 2000, - p.117.

## Claims

1. crystal modification of 6-hydroxy-4-oxo-2,4-dihydro-1H-pyrrolo[3,2,1-ij]-quinoline-5-carboxylic acid para-methoxyanilide with the following characteristic interplanar spacings (d) and relative intensities of reflections (Iᵣₑₗ)
| d, Å - Iᵣₑₗ | d, Å -Iᵣₑₗ | d, Å - Iᵣₑₗ |
|---|---|---|
| 15.189 - 6 | 3.868 - 1 | 2.776 -<1 |
| 11.176 - 54 | 3.753 - 4 | 2.740 -<1 |
| 7.912 - 5 | 3.728 - 10 | 2.728 - 1 |
| 7.821 - 10 | 3.694 - 52 | 2.718 - 1 |
| 7.750 - 13 | 3.686 - 10 | 2.656 - 1 |
| 7.634 - 4 | 3.650 - 4 | 2.642 - 1 |
| 7.426 - 28 | 3.600 - 4 | 2.630 - <1 |
| 7.376 - 39 | 3.575 - 6 | 2.601 - <1 |
| 6.751 - 18 | 3.546 - 42 | 2.585 - 1 |
| 6.674 - 36 | 3.520 - 7 | 2.576 - <1 |
| 6.027 - <1 | 3.475 - <1 | 2.494 - <1 |
| 5.867 - 4 | 3.442 - 1 | 2.482 - 1 |
| 5.798 - 11 | 3.417 - <1 | 2.476 - 1 |
| 5.718 - 21 | 3.388 - 2 | 2.446 - <1 |
| 5.648 - 15 | 3.362 - 24 | 2.412 - <1 |
| 5.609 - 10 | 3.339 - 100 | 2.402 - 1 |
| 5.561 - 3 | 3.323 - 31 | 2.312 - <1 |
| 5.084 - 4 | 3.257 - 2 | 2.301 - <1 |
| 4.907 - 1 | 3.240 - 3 | 2.295 -<1 |
| 4.856 - <1 | 3.220 - 2 | 2.283 - 1 |
| 4.751 - 2 | 3.183 - 1 | 2.273 - 1 |
| 4.714 - 5 | 3.145 - 2 | 2.267 - <1 |
| 4.681 - 7 | 3.108 - 17 | 2.247 - <1 |
| 4.660 - 6 | 3.083 - 2 | 2.236 - 1 |
| 4.589 - 1 | 3.071 - <1 1 | 2.227 - <1 |
| 4.567 - 3 | 3.049 - <1 | 2.221 - <1 |
| 4.548 - 5 | 2.966 - <1 | 2.166 - <1 |
| 4.356 - <1 | 2.955 - 1 | 2.113 - <1 |
| 4.225 - 1 | 2.944 - 1 | 2.103 - 2 |
| 4.187 - 4 | 2.933 - <1 | 2.095 - <1 |
| 4.168 - 8 | 2.869 - <1 | 2.086 - <1 |
| 4.140 - 13 | 2.854 - <1 | 2.072 - <1 |
| 4.100 - 4 | 2.840 - <1 | 2.051 - <1 |
| 4.083 - 2 | 2.831 - 1 | 2.043 - 1 |
| 4.047 - 1 | 2.813 - 1 | 2.037 - <1 |
| 3.992 - 1 | 2.806 - 1 | 2.021 - <1 |
| 3.977 - 4 | 2.793 - 2 | 1.880 - <1 |
| 3.946 - 12 | 2.783 - 1 | 1.874 - <1 |
| 3.907 - 7 | | |
that exhibits diuretic activity.

## Patentansprüche

1. Kristallmodifikation des 6-Hydroxy-4-oxo-2,4-dihydro-1H-pyrollo[3,2,1-ij}-chinolin-5-carbonsäure-para-methoxyanilids mit den folgenden, charakteristischen, interplanaren Abständen (d) und relativen Reflexionsstärken (Iᵣₑₗ)
| d, Å - Iᵣₑₗ | d, Å - Iᵣₑₗ | d, Å *-* Iᵣₑₗ |
|---|---|---|
| 15.189 - 6 | 3.868 - 1 | 2.776 - <1 |
| 11.176 - 54 | 3.753 - 4 | 2.740 - <1 |
| 7.912 - 5 | 3,728 - 10 | 2.728 - 1 |
| 7.821 - 10 | 3.694 - 52 | 2.718 - 1 |
| 7.750 - 13 | 3.666 - 10 | 2.656 - 1 |
| 7.634 - 4 | 3.650 - 4 | 2.642 -1 |
| 7.426 - 28 | 3.600 - 4 | 2.630 -<1 |
| 7.376 - 39 | 3.575 - 8 | 2.601 -<1 |
| 6.751 - 18 | 3.546 - 42 | 2.585 -1 |
| 6.674 - 36 | 3.620 - 7 | 2.576 - <1 |
| 6.027 - <1 | 3.475 - <1 | 2.494 - <1 |
| 5.867 - 4 | 3.442 - 1 | 2.482 - 1 |
| 6.798 - 11 | 3.417 - <1 | 2.475 - 1 |
| 5.718 - 21 | 3.388 - 2 | 2.446 - <1 |
| 5.648 - 15 | 3.362 - 24 | 2.412 - <1 |
| 6.609 - 10 | 3.339 - 100 | 2.402 - 1 |
| 5.561 - 3 | 3.323 - 31 | 2.312 - <1 |
| 5.084 - 4 | 3.257 - 2 | 2.301 - <1 |
| 4.907 - 1 | 3.240 - 3 | 2.295 -<1 |
| 4.856 - <1 | 3.220 - 2 | 2.283 - 1 |
| 4.751 - 2 | 3.183 - 1 | 2.273 - 1 |
| 4.714 - 6 | 3.145 - 2 | 2.267 - <1 |
| 4.681 - 7 | 3.108 - 17 | 2.247 - <1 |
| 4.660 - 6 | 3.083 - 2 | 2.236 - 1 |
| 4.589 - 1 | 3.071- <1 | 2.227 - <1 |
| 4.567 - 3 | 3.049 - <1 | 2.221 - <1 |
| 4.548 - 5 | 2.966 - <1 | 2.166 - <1 |
| 4.356 - <1 | 2.955 - 1 | 2.113 - <1 |
| 4.225 - 1 | 2.944 - 1 | 2.103 - 2 |
| 4.187 - 4 | 2.933 - <1 | 2.095 - <1 |
| 4.168 - 8 | 2.869 - <1 | 2.086 - <1 |
| 4.140 - 13 | 2.854 - <1 | 2.072 - <1 |
| 4.100 - 4 | 2.840 - <1 | 2.061 -<1 |
| 4.083 - 2 | 2.831 - 1 | 2.043 - 1 |
| 4.047 - 1 | 2.813 - 1 | 2.037 - <1 |
| 3.992 - 1 | 2.806 - 1 | 2.021 - <1 |
| 3.977 - 4 | 2.793 - 2 | 1.880 - <1 |
| 3.946 - 12 | 2.783 - 1 | 1.874 - <1 |
| 3.807 - 7 | | |
wodurch eine harntreibende Aktivität vermieden wird.

## Revendications

1. Forme cristalline du para-méthoxyanilide de l'acide 6-hydroxy-4-oxo-2,4-dihydro-1H-pyrrolo[3,2,1-ij]quinoléine-5-carboxylique avec les espacements interplanaires caractéristiques suivants (d) et les intensités de réflexion relatives (Iᵣₑₗ) suivantes :
| d, Å -Iᵣₑₗ | d, Å - Iᵣₑₗ | d, Å Iᵣₑₗ |
|---|---|---|
| 15.189 - 6 | 3.868 - 1 | 2.776 -<1 |
| 11.176 - 54 | 3.753 - 4 | 2.740 -<1 |
| 7.912 - 5 | 3.723 - 10 | 2.728 - 1 |
| 7.821 - 10 | 3.694 - 52 | 2.718 - 1 |
| 7.750 - 13 | 3.666 - 10 | 2.656 - 1 |
| 7.634 - 4 | 3.850 - 4 | 2.642 - 1 |
| 7.426 - 28 | 3.600 - 4 | 2.630 -<1 |
| 7.378 - 39 | 3.575 - 8 | 2.601 -<1 |
| 6.751 - 8 | 3.546 - 42 | 2.585 - 1 |
| 6.674 - 36 | 3.520 - 7 | 2.576 - <1 |
| 6.027 - <1 | 3.475 - <1 | 2.494 - <1 |
| 5.867 - 4 | 3.442 - 1 | 2.482 - 1 |
| 5.798 - 11 | 3.417 - <1 | 2.475 - 1 |
| 5.78 - 21 | 3.388 - 2 | 2.446 - <1 |
| 5.648 - 15 | 3.362 - 24 | 2.412 - <1 |
| 5.609 - 10 | 3.339 - 100 | 2.402 - 1 |
| 5.561 - 3 | 3.323 - 31 | 2.312 - <1 |
| 5.084 - 4 | 3.257 - 2 | 2.301 - <1 |
| 4,907 - 1 | 3.240 - 3 | 2.295 - <1 |
| 4.856 - <1 | 3.220 - 2 | 2.283 - 1 |
| 4.751 - 2 | 3.163- 1 | 2.273 - 1 |
| 4.714 - 6 | 3.145 - 2 | 2.267 - <1 |
| 4.681 - 7 | 3.108 - 17 | 2.247 - <1 |
| 4.660 - 6 | 3.083 - 2 | 2.236 - 1 |
| 4.589 - 1 | 3.071 - <1 | 2.227 - <1 |
| 4.567 - 3 | 3.049 - <1 | 2.221 - <1 |
| 4.548 - 5 | 2.966 - <1 | 2.166 - <1 |
| 4.356 - <1 | 2.955 - 1 | 2.113 - <1 |
| 4.225 - 1 | 2.944 - 1 | 2.103 - 2 |
| 4.187 - 4 | 2.933 - <1 | 2.095 - <1 |
| 4.168 - 8 | 2.869 - <1 | 2.086 - <1 |
| 4.140 - 13 | 2.854 - <1 | 2.072 - <1 |
| 4.100 - 4 | 2.840 - <1 | 2.051 -<1 |
| 4.083 - 2 | 2.831 - 1 | 2.043 - 1 |
| 4.047 - 1 | 2.813 - 1 | 2.037 - <1 |
| 3.992 - 1 | 2.808 - 1 | 2.021 - <1 |
| 3.977 - 4 | 2.793 - 2 | 1.880- <1 |
| 3.946 - 12 | 2.783 - 1 | 1.874 - <1 |
| 3.907 - 7 | | |
qui présentent une activité diurétique.
